# EUROPÄISCHE PATENTANMELDUNG

(11) **EP 1 721 624 A1**
(43) Veröffentlichungstag der Anmeldung: **15.11.2006**
(21) Anmeldenummer: 06009168.3
(22) Anmeldetag: 03.05.2006
(51) Int. Cl.: A61L 27/48

(54) **Verfahren zur Herstellung von bioresorbierbaren Verbundmaterialien und seine Verwendung als Implantatmaterial**

(30) Priorität: 09.05.2005 DE 102005022176
(71) Anmelder: Martin-Luther-Universität Halle-Wittenberg, 06108 Halle/Saale (DE); UFZ Umweltforschungszentrum Leipzig-Halle GmbH, 04318 Leizig (DE)
(72) Erfinder: Henning, Sven, 06114 Halle/Saale (DE); Kim, Gyeong-Man, 04249 Leipzig (DE); Michler, Georg Hannes, 06179 Langenbogen (DE); Wendtlandt, Karin-Dagmar, 04105 Leipzig (DE); Jeschorek, Mirko, 04347 Leipzig (DE); Mirschel, Gabriele, 04229 Leipzig (DE)
(74) Vertreter: Wissenbach, Gisela

(57) **Zusammenfassung**

Die Erfindung beschreibt ein Verfahren zur Herstellung von bioresorbierbaren Verbundmaterialien umfassend eine Polymermatrix aus Poly-β-hydroxybuttersäure (PHB) mit einem Molekulargewicht von 600.000 bis 2.300.000 g/mol und Hydroxylapatit-Nanopartikeln bis zu 500 nm mit hohen Füllgraden bis 60%, vorzugsweise für den Einsatz als lmplantatmaterial. Das Material ist insbesondere geeignet für lasttragende Anwendungen in der Orthopädie und der Kieferheilkunde.

## Beschreibung

Die Erfindung beschreibt ein Verfahren zur Herstellung von bioresorbierbaren Verbundmaterialien umfassend eine Polymermatrix aus Poly-β-hydroxybuttersäure (PHB) mit einem Molekulargewicht von 600.000 bis 2.300.000 g/mol und Hydroxylapatit-Nanopartikeln bis zu 500 nm mit hohen Füllgraden, vorzugsweise für den Einsatz als Implantatmaterial. Das Material ist insbesondere geeignet für lasttragende Anwendungen in der Orthopädie und der Kieferheilkunde.

Besonderes Interesse gilt gegenwärtig Implantaten aus biodegradierbaren Polymeren und Keramiken. Resorbierbare Polymere werden zur Fixation von Knochenfrakturen bereits klinisch eingesetzt. Biodegradable Implantate, die sich nach Erfüllung ihrer Funktion im Körper auflösen, erübrigen einen zweiten operativen Eingriff, mit dem die Fixationselemente, wenn sie aus Stahl oder Titan bestehen, wieder entfernt werden müssen. Sie sind deshalb sowohl in Hinsicht auf die Entlastung des betroffenen Patienten als auch unter dem Aspekt der Kostenreduzierung von großer Bedeutung.

Auch für den Knochenersatz haben diese Entwicklungen große Bedeutung. Resorbierbare Folien aus Verbundmaterialien zur Verbesserung der Knochendefektbehandlung in der Kieferchirurgie sind dafür ein Beispiel. Darüber hinaus gilt als gesichert, dass mineralische Füllkomponenten, z.B. Hydroxylapatit-Partikel, die gerichtete Neubildung des Knochens stimulieren.

Verfahren zur Einarbeitung von Hydroxylapatit (HAp) in Form von mikro- und nanoskopischen Partikeln in eine polymere Matrix wie z.B. Polyethylen, Polyester, Polylactide u.a. sind bekannt. So beschreiben Bonfield et al. (EP 0 049 720 A1) die thermomechanische Einarbeitung des Füllstoffes in das Polymer. Es werden Partikelgrößen zu 90% < 100 pm bis 0,05 Nm verwendet. Auf diese Weise werden Kompositmaterialien erzeugt, deren Steifigkeit höher ist als die des als Ausgangsmaterial verwendeten Polymerwerkstoffes. Die Einarbeitung der Partikel führt zu einer verbesserten Biokompatibilität in dem Sinne, dass der in der inerten Matrix eingeschlossene Füllstoff die Knochenneubildung stimulieren kann. Demzufolge wird eine Verwendung der so hergestellten Kompositwerkstoffe als Biomaterial für die Orthopädie vorgeschlagen. Die dort beschriebenen Implantate sind als Dauerimplantate konzipiert, die eine den metallischen Implantaten überlegene Biokompatibilität aufweisen.

Gegenwärtig ermöglichen die Komposite mit bioinerten Matrixpolymeren jedoch nur die Zugänglichkeit von an der Materialoberfläche verfügbaren Füllstoffen. Eine dauerhafte und gleichmäßige Entfaltung der bioaktiven Wirkung ist daher in Frage gestellt. Ein großes Problem stellt insbesondere die Bildung von Agglomeraten des Füllstoffs in der Matrix bei hohem Mengenanteil dar.

Der Erfindung lag deshalb die Aufgabe zugrunde, geeignete bioresorbierbare Polymerverbundmaterialien mit verbesserter Biokompatibilität zu entwickeln, die einen hohen Füllgrad der mineralischen Komponente Hydroxylapatit aufweisen, um so einen breiteren Einsatz als Implantate ermöglichen. Die Aufgabe der Erfindung bestand weiterhin darin, ein ökonomisches Verfahren zu deren Herstellung zu entwickeln, wobei insbesondere Verbundmaterialien hergestellt werden sollen, die bei hohen Füllgraden eine homogene Verteilung des Füllstoffes zeigen.

Die Aufgabe wird durch ein Verfahren zur Herstellung feinverteilter Nanokompositwerkstoffe mit hohen Füllgraden gelöst, wobei in eine Polymermatrix aus Poly-ß-hydroxybuttersäure (PHB) mit einem hohen Molekulargewicht von 600.0008/mol bis 2.300.000 g/mol der Füllstoff Hydroxylapatit in Form von Nanopartikeln mit einer Partikelgröße bis 500 nm eingebracht wird.

Bevorzugt wird eine Poly-β-hydroxybuttersäure eingesetzt, die durch einen methanotrophen Bakterienstamm produziert wird. Ganz besonders, bevorzugt handelt es sich um eine Poly-β-hydroxybuttersäure die durch den Bakterienstamm *Methylocystis spec.* DSM 7674 produziert wird. Der Stamm ist bei der Deutschen Sammlung für Mikroorganismen und Zeltkulturen, Braunschweig als Patentstamm hinterlegt und wurde am 21.04.2005 entsprechend verlängert. Die Herstellung Poly-β-hydroxybuttersäure ist in DE 196 19 084 C2 beschrieben. Sie zeichnet sich durch ein hohes Molekulargewicht 600.000 bis 2.300.000 g/mol und eine hohe Reinheit (Stickstoffgehalt <0,02%) aus. Die bevorzugt verwendete Poly-ß-hydroxybuttersäure ist auch als aus Methan gewonnene hochmolekulare Poly-3-hydroxybuttersäure unter dem Namen Methanomer0 bekannt.

Gemäß vorliegender Erfindung wird die Poly-ß-hydroxybuttersäure als Trockenmasse, z.B. in Pulverform oder bereits aus der Biomasse in Lösung, indem bei der üblichen Extraktion auf eine nachfolgende Ausfällung verzichtet wird, eingesetzt. Die Techniken richten sich nach der Verwendung, wobei jede Verfahrensvariante bestimmte anwendungsorientierte Vorteile hat.

Die erfindungsgemäßen Verbundmaterialien werden durch Einbringen der Hydroxylapatit-Nanopartikel mit einer Partikelgröße von 10 nm bis 500 nm, vorzugsweise mit Nanopartikeln mit Größen < 100 nm, hergestellt. Besonders bevorzugt sind Partikelgrößen von 10 bis 50 nm.

Die Hydroxylapatit-Nanopartikel werden via Lösungprozess in die Polymermatrix eingebracht. Die Hydroxylapatit-Nanopartikel werden in einer wässrigen Lösung suspendiert und anschließend in die Polymerlösung, die aus Polyhydroxybuttersäure-Trockenmasse durch Lösen in einem Lösungsmittel ggf. unter Erhitzen hergestellt wurde, gegeben. Das Verfahren wird vorzugsweise im Ultraschallbad unter Erhitzen durchgeführt bis die Lösung transparent ist.

Alternativ können die in wässriger Lösung suspendierten Hydroxylapatit-Nanopartikel auch direkt in eine Polyhydroxybuttersäure-Lösung gegeben werden. Vorzugsweise wird die Polyhydroxybuttersäure aus der Biomasse in Lösung extrahiert, indem die vorgewaschene Biomasse mit einem Lösungsmittel versetzt, ggf. erhitzt und anschließend filtriert wird. Ein Ausfällen der Polyhydroxybuttersäure entfällt.

Als Lösungs- und Extraktionsmittel werden vorzugsweise Dichlorethan und/oder Chloroform eingesetzt.

Je nach Anwendungsziel wird z.B. das Lösungsmittels abgedampft und es werden die Materialien bevorzugt in Form von dünnen flexiblen Filmen erhalten, die eine feine Verteilung des nanoskopischen Füllstoffes im Matrixpolymer zeigen. Alternativ können mittels Elektrospinnverfahren Hybrid-Nanofasern hergestellt werden. Ggf. können die durch Elektrospinnen gewonnenen Fasern einer Heißkompaktierung unterzogen werden, wodurch ebenfalls ein kompakter Festkörper erreicht wird.

Die Herstellung von Polymer-Nanofasern durch Elektrospinn-Verfahren ist dem Fachmann bekannt. Z.B. ist in DE 100 40 897 A1 die Herstellung von Fasern mit Durchmessern von 20 bis 4000 nm beschrieben.

Mit der vorliegenden Erfindung wird ein Verfahren zur Verfügung gestellt, das ein echtes Nanokomoposit mit einer biokompatiblen Matrix und Füllstoffpartikeln im Größenbereich 10 bis 500 nm erzeugt. Mit dem erfindungsgemäßen Verfahren können hohe Füllgrade bis zu 60 Masse% erreicht werden. Die Einarbeitung der Füllstoffpartikel (HAp) erfolgt so, dass die Teilchen einzeln und gut in der Polymermatrix aus PHB dispergiert vorliegen, eine Agglomeration wird vermieden.
Die Verwendung der PHB bietet besondere Vorteile, denn beim Abbau dieses speziellen Biopolymers PHB entsteht die für den Menschen ohne Probleme verträgliche Buttersäure. Andere Ersatzstoffe aus anderen Materialien haben sich gerade beim Abbauprozess als schwer verträglich herausgestellt, da z.B. entstehende Milchsäure die Heilung verzögert. Dagegen ermöglicht die vollständige Resorption des Matrixpolymers (PHB) den schrittweisen Zugang zum bioaktiven Füllstoff HAp. Im Zuge der Degradation wird die bioaktive, die Knochenneubildung anregende Komponente schrittweise vollständig verfügbar. Die bioaktive Wirkung bleibt bis zur vollständigen Resorption des Implantates erhalten. Der verwendete Füllstoff selbst wird im Körper vollständig resorbiert.

Der beschriebene Werkstoff besteht bevorzugt aus der biokompatiblen, resorbierbaren Polymermatrix hochmolekularer auf der Basis von Methan hergestellter Poly-3-hydroxybuttersäure (HMW-PHB, Methanomer^{R}), und dem bioaktiven Füllstoff Hydroxylapatit. Durch das beschriebene Verfahren wird unter Einsatz dieser speziellen, hochmolekularen Poly-3-hydroxybuttersäure (HMW-PHB) in Kombination mit den beschriebenen Verarbeitungstechniken eine nanoskopische und homogene Verteilung der Hydroxylapatit-Komponente mit hohen Füllgraden erreicht.

Die für die hier beschriebenen Komposite ermittelten mechanischen Kennwerte sind insbesondere bedingt durch:
1. das bevorzugt verwendete Polymer PHB mit den sehr hohen Molekulargewichten von 600.000 g/mol bis 2.300.000 g/mol,
2. den verwendeten bioaktiven Füllstoff Hydroxylapatit mit bevorzugten Partikelgrößen kleiner 100 nm, welcher fein dispergiert werden kann und keine Agglomerate bildet,
3. die Beeinflussung des verwendeten Füllstoffs auf das Kristallisationsverhalten des sehr gut kristallisationsfähigen Matrixpolymers, wobei die Neigung zur Bildung einer grobsphärolithischen Struktur unterdrückt wird und damit verbundene sprödartige Versagensmechanismen (inter- und intrasphärolithische Risseinleitung) vermieden werden.

Dieses führte insgesamt zu neuartigen mikromechanischen Mechanismen, die zur erhöhten Festigkeit und Zähigkeit des erfindungsgemäßen Materials führen. Das erfindungsgemäß hergestellte Verbundmaterial ist deshalb insbesondere zum temporären Ersatz von Knochen und zum Auffüllen von Knochendefekten geeignet, z.B. nach Entfernung eines Tumors, sowie zur Fixierung bei Frakturen, bei Kallusdistraktion und anderen Verfahren der wiederherstellenden und kosmetischen Chirurgie. Im Gegensatz zu bisher verwendeten metallischen Implantaten kann eine zweite Operation zur Entfernung der implantierten Vorrichtung entfallen, da beide Komponenten des Kompositwerkstoffes im menschlichen Körper vollständig resorbiert werden. Die Degradationsprodukte werden in natürliche Stoffwechselprozesse eingebunden, sind nicht toxisch und nicht kanzerogen. Das beschriebene Material ist biokompatibel, und zwar sowohl im Sinne einer strukturellen Kompatibilität als auch einer Oberflächenkompatibilität. Unter struktureller Kompatibilität wird hier verstanden, dass die Morphologie des Kompositwerkstoffes die Nanostruktur des Knochens nachahmt, wodurch die Festigkeit und die Steifigkeit des gesunden kompakten Knochens erreicht werden. Insbesondere die Anpassung des Elastizitätsmoduls an die vom kortikalen Knochen vorgegebenen Werte von 12 bis 18 GPa (X.E. Guo in: Bone mechanics handbook, S.C. Cowin, 2nd edition, CRC press, Boca Raton 2001, Chapt.10) ist von überragender Bedeutung, da so eine Atrophie bzw. übermäßige Resorption des *Knochens durch ein so genanntes stress shielding, also die Entlastung des* Knochengewebes durch die vollständige Überleitung mechanischer Spannungen in das Implantat, vermieden werden kann. Die erreichte Festigkeit des Kompositwerkstoffes gewährleistet den Einsatz auch in lasttragenden Bereichen, der aufgrund der Sprödigkeit bisher vorhandener Polyhydroxyalkanoate bzw. von Kompositen aus Polyhydroxyalkanoaten und Füllstoffen, insbesondere bioaktiven Keramiken und Gläsern, nicht gewährleistet ist. Unter Oberflächenkompatibilität wird verstanden, dass die im Komposit vorhandenen Komponenten, also das Matrixpolymer PHB und der Füllstoff, nicht toxisch, nicht allergen und nicht kanzerogen sind.

Nachfolgend soll die Erfindung an Ausführungsbeispielen näher erläutert werden.

Ausführungsbeispiele

### Beispiel 1:

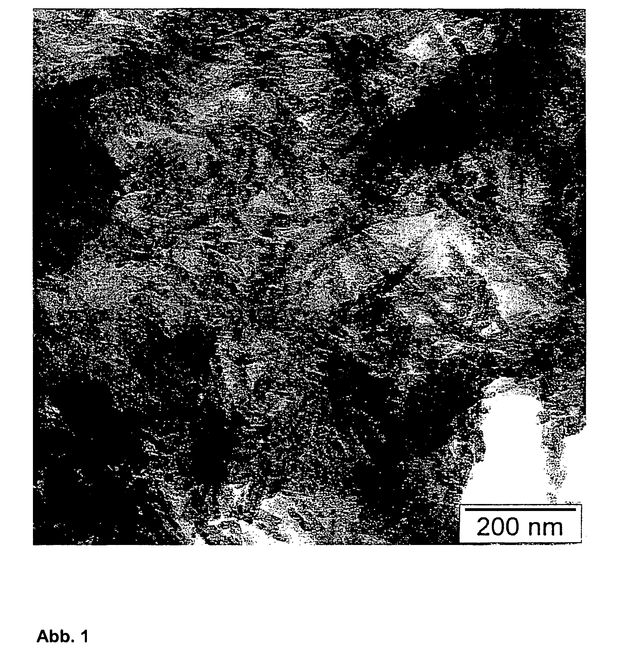

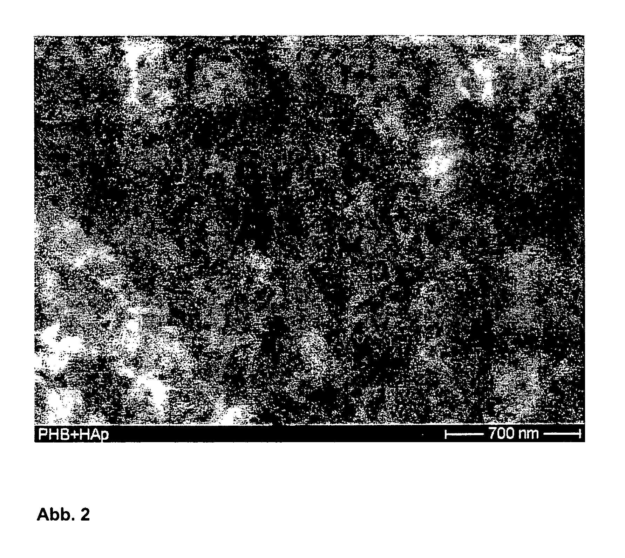

0,5 g einer Paste aus Hydroxylapatit-Nanopartikeln (OSTIM^{R}-SPR, HERAEUS, Deutschland) werden in 1 ml destilliertem Wasser gegeben. Die Mischung wird so lange bei Raumtemperatur in einem Ultraschallbad belassen, bis die Suspension völlig transparent ist (ca. 1 h). Diese Suspension wird in 5 ml einer ca. 10%igen Lösung des Polymers PHB Methanomer^{R} mit Mw=1.200.000 g/mol in Chloroform gegeben. Die Polymerlösung wird zuvor wie folgt hergestellt: 1 g Polyhydroxybutyrat-Trockenmasse werden in 10 ml Chloroform bei 60°C in einem Ultraschallbad gelöst. Die Mischung aus Polymerlösung und Hydroxylapatit-Suspension wird bei 80°C ebenfalls im Ultraschallbad belassen, bis die Lösung vollständig transparent ist (ca. 4h). Aus der Lösung werden Filme gegossen. Nach Abdampfen des Lösungsmittels erhält man einen ca. 100 Nm dicken, flexiblen Film. Transmissions- bzw. rasterelektronenmikroskopische Aufnahmen an Ultradünnschnitten bzw. Bruchflächen dieses Filmes zeigen eine feine Verteilung des nanoskopischen Füllstoffes im Matrixpolymer, wie aus den Abb. 1 und 2 ersichtlich.

Abb. 1: Transmissionselektronenmikroskopische Aufnahme eines im Lösungsverfahren hergestellten Komposites aus Methanomer und nanoskopischen HAp-Partikeln. Die dunkel erscheinenden, lanzettförmigen Nanopartikel sind gut in der Polymermatrix dispergiert.

Abb. 2: Die rasterelektronenmikroskopische Aufnahme (ESEM, Rückstreuelektronen-Materialkontrast) einer Bruchfläche des im Lösungsverfahren hergestellten Komposites zeigt hell erscheinende Nanokristallite, die über einen großen Bereich gleichmäßig in der PHB-Matrix dispergiert sind.

### Beispiel 2:

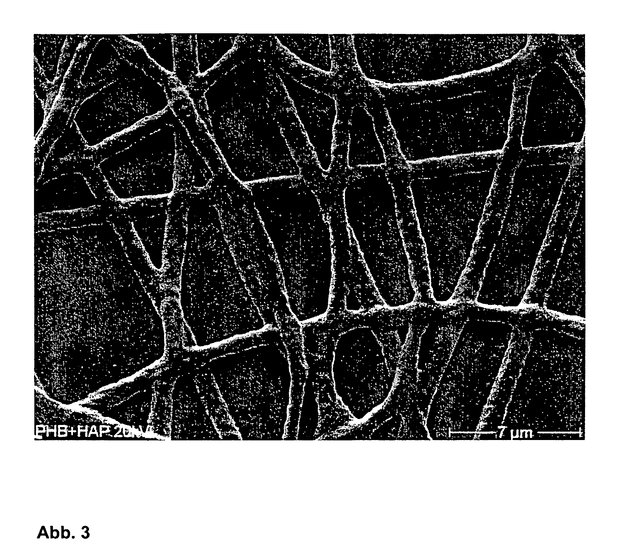

0,5 g einer Paste aus Hydroxylapatit-Nanopartikeln (OSTIM^{R}-SPR, HERAEUS, Deutschland) werden in 1 ml destilliertem Wasser gegeben. Die Mischung wird so lange bei Raumtemperatur in einem Ultraschallbad belassen, bis die Suspension völlig transparent ist (ca. 1 h). Diese Suspension wird in 5 ml einer ca. 10%igen Lösung des Polymers PHB Methanomer^{R}mit Mw=1.200.000 g/mol in Chloroform gegeben. Die Polymerlösung wird zuvor wie folgt hergestellt: 1 g Polyhydroxybutyrat-Trockenmasse werden in 10 ml Chloroform bei 60°C in einem Ultraschallbad gelöst. Die Mischung aus Polymerlösung und Hydroxylapatit-Suspension wird bei 80°C ebenfalls im Ultraschallbad belassen, bis die Lösung vollständig transparent ist (ca. 4h). Die den nanoskopisch dispergierten Hydroxylapatit enthaltende Polymerlösung wird in einem Elektrospinnverfahren zu nanoskopischen Faser versponnen. Die angelegte Spannung beträgt 0 bis 30 kV, die bevorzugte Spannung beträgt 8 bis 12 kV. Die Elektroden sind in einem Abstand von 10 bis 200 mm, bevorzugt 40 mm angeordnet. Die gewonnenen Fasern haben Durchmesser von 50 bis 1000 nm (s. Abb. 3: Rasterelektronenmikroskopische Aufnahme (ESEM) der durch Elektrospinnen hergestellten hybriden Nanofasern).

### Beispiel 3:

Nanokomposite aus PHB und HAp wurden durch Heißkompaktieren der zuvor wie in Beispiel 2 durch Elektrospinnen erzeugte Hybrid-Nanofasern hergestellt. Die Temperatur liegt in einem Bereich zwischen 5 und 30 K oberhalb des Schmelzpunktes des teilkristallinen PHB, bevorzugt zwischen 10 und 20 K oberhalb des genannten Schmelzpunktes (Schmelztemperatur Tm ca. 180°C).

### Beispiel 4:

Der PHB-Syntheseprozeß wird auf herkömmliche Art mit einem Methan/Luft-Gemisch durchgeführt, wobei ein PHB-Gehalt der Biomasse von 39% erzielt wird. Die Isolierung der PHB wird nicht bis zum Ende (Gewinnung von PHB in Pulverform) vollzogen, sondern nach gewöhnlichen Vorwäschen direkt nach der Extraktion mit einem geeigneten Lösungsmittel ohne Ausfällung abgebrochen. Dazu werden 5g vorgewaschene Biomasse mit 25 ml Chloroform versetzt, 3 h im Wasserbad unter Rühren bei 50°C extrahiert und anschließend filtriert (Whatman-Filterpapier Nr. 4). So wird eine Lösung erhalten, deren Konzentration an PHB 10,8 g/l beträgt. Damit liegt die Ausbeute bei 38,5%. Die Lösung ist bei 4°C mindestens 6 Monate stabil und sehr rein (Stickstoffgehalt <_ 0,03%). Diese Lösung kann direkt für alle beschriebenen Methoden zur Einbringung der HAp-Nanopartikel eingesetzt werden.

## Patentansprüche

1. Verfahren zur Herstellung von bioresorbierbaren Verbundmaterialien umfassend eine Polymermatrix und Hydroxylapatit-Nanopartikel mit hohen Füllgraden, **dadurch gekennzeichnet, dass** in eine Polymermatrix aus Poly-ßhydroxybuttersäure (PHB) mit einem Molekulargewicht von 600.000g/mol bis 2.300.000 g/mol Hydroxylapatit in Form von Nanopartikeln mit Partikelgrößen bis zu 500 nm eingebracht wird.

2. Verfahren nach Anspruch 1, **dadurch gekennzeichnet, dass** die eingesetzte PHB durch einen methanotrophen Bakterienstamm produziert wird.

3. Verfahren nach Anspruch 1 oder 2, **dadurch gekennzeichnet, dass** die eingesetzte PHB durch den methanotrophen Bakterienstamm *Methylocystis* spec. DSM 7674 produziert wird.

4. Verfahren nach einem der Ansprüche 1 bis 3, **dadurch gekennzeichnet, dass** die eingesetzte PHB als Trockenmasse (in Pulverform) oder in Lösung direkt aus der Biomasse eingesetzt wird.

5. Verfahren nach einem der Ansprüche 1 bis 4, **dadurch gekennzeichnet, dass** die Hydroxylapatit- Nanopartikel in einer Größe von 10 bis 500 nm, vorzugsweise in einer Größe< 100 nm eingesetzt werden.

6. Verfahren nach einem der Ansprüche 1 bis 5, **dadurch gekennzeichnet, dass** die Hydroxylapatit-Nanopartikel via Lösungprozess in die Polymermatrix eingebracht werden, wobei die Hydroxylapatit-Nanopartikel in einer wässrigen Lösung suspendiert werden und in eine Polymerlösung, die aus PHB-Trockenmasse durch Lösen in einem Lösungsmittel hergestellt wird, gegeben werden.

7. Verfahren nach einem der Ansprüche 1 bis 5, **dadurch gekennzeichnet, dass** die Hydroxylapatit-Nanopartikel in die Polymermatrix eingebracht werden, indem die Hydroxylapatit-Nanopartikel in einer wässrigen Lösung suspendiert werden und direkt in eine Polyhydroxybuttersäure-Lösung gegeben werden.

8. Verfahren nach Anspruch 7, **dadurch gekennzeichnet, dass** die PHB-Lösung aus der Biomasse durch Extraktion hergestellt wird.

9. Verfahren nach einem der Ansprüche 1 bis 8, **dadurch gekennzeichnet, dass** von der PBH-Lösung mit den dispergierten Hydroxylapatit-Partikeln das Lösungmittel abgedampft und das Verbundmaterial in Form von dünnen Filmen gewonnen wird.

10. Verfahren nach einem der Ansprüche 1 bis 8, **dadurch gekennzeichnet, dass** die PBH-Lösung mit den dispergierten Hydroxylapatit-Partikeln mittels Elektrospinnverfahren zu einem Verbundmaterial in Form von Nanofasern versponnen wird.

11. Verfahren nach Anspruch 10, **dadurch gekennzeichnet, dass** die durch Elektrospinnen gewonnenen Fasern einer Heißkompaktierung unterzogen werden.

12. Verfahren nach einem der Ansprüche 1 bis 11, **dadurch gekennzeichnet, dass** das Lösungs- und Extraktionsmittel Dichlorethan und/oder Chloroform ist.

13. Bioresorbierbare Verbundmaterialien umfassend eine Polymermatrix und Hydrxylapatit-Nanopartikel mit hohen Füllgraden hergestellt nach einem der Ansprüche 1 bis 11.

14. Verwendung von bioresorbierbaren Verbundmaterialien gemäß Anspruch 13 als Implantatmaterial.

15. Verwendung nach Anspruch 14, **dadurch gekennzeichnet, dass** es in der Orthopädie und Kieferheilkunde verwendet wird.
